# EUROPÄISCHE PATENTSCHRIFT

(11) **EP 3 484 424 B1**
(45) Veröffentlichungstag und Bekanntmachung des Hinweises auf die Patenterteilung: **08.07.2020**
(21) Anmeldenummer: 17740017.3
(22) Anmeldetag: 17.07.2017
(51) Int. Cl.: A61F 9/008

(54) **SYSTEM ZUR AUGENTHERAPIE MITTELS GEWEBEBEARBEITUNG DURCH NICHTLINEARE WECHSELWIRKUNG**
SYSTEM FOR THERAPY OF THE EYE BY TREATING TISSUE USING NON-LINEAR INTERACTION
SYSTÈME DE TRAITEMENT OCULAIRE PAR TRAITEMENT DE TISSU PAR INTERACTION NON LINÉAIRE

(30) Priorität: 18.07.2016 DE 102016213095; 12.04.2017 DE 102017107915
(43) Veröffentlichungstag der Anmeldung: 22.05.2019
(73) Patentinhaber: Carl Zeiss Meditec AG, 07745 Jena (DE)
(72) Erfinder: NOBIS, Thomas, 07743 Jena (DE); HANFT, Marco, 07743 Jena (DE); BISCHOFF, Mark, 07749 Jena (DE)
(74) Vertreter: Patentanwälte Geyer, Fehners & Partner mbB
(86) Internationale Anmeldenummer: PCT/EP2017/068044
(87) Internationale Veröffentlichungsnummer: WO 2018/015349

(56) Entgegenhaltungen:
- EP-A1- 1 792 593
- WO-A1-2007/124602
- WO-A1-2008/077532
- WO-A2-03/057100
- WO-A2-2011/059958
- DE-A1-102008 011 836

## Beschreibung

Die Erfindung bezieht sich auf ein System zur Augentherapie mittels Bearbeitung von Gewebe durch nichtlineare Wechselwirkung mit Therapiestrahlung, wobei das System aufweist: eine Lasereinrichtung, welche die Therapiestrahlung bereitstellt, eine Fokussiereinrichtung, die ein Bildfeld, das in einem Therapievolumen liegt, aufweist und die Therapiestrahlung derart zur einem Fokus im Bildfeld bündelt, dass innerhalb des Gewebe die Bearbeitung durch nichtlineare Wechselwirkung erzeugbar ist, eine der Fokussiereinrichtung vorgeordnete xy-Scannereinrichtung zur lateralen Verschiebung des Fokus im Bildfeld, eine z-Scannereinrichtung, die eine Tiefenlage des Fokus im Therapievolumen verstellt, und eine Steuereinrichtung, welche den xy-Scanner und den z-Scanner ansteuert.

Beispiele für die Anwendung von Systemen zur Augentherapie sind die lasergestützte refraktive Augenchirurgie oder die lasergestützte Katarakt-Operation. Bei der refraktiven Augenchirurgie werden mittels Laserstrahlung innerhalb der Kornea Schnittflächen erzeugt, die ein Volumen isolieren und entfernbar machen. Das Volumen ist so bemessen, dass seine Entfernung die Korneakrümmung auf eine Weise ändert, die eine zuvor bestandene Fehlsichtigkeit ausgleicht. Bei der Katarakt-Operation wird die natürliche, opak gewordene Augenlinse durch eine künstliche Intraokularlinse (IOL) ersetzt. Dazu wird in den Kapselsack der Augenlinse an seiner Vorderseite ein Loch geschnitten. Durch dieses Loch wird die Linse nach vorheriger Fragmentierung entfernt und eine künstliche Intraokularlinse (IOL) eingesetzt. Für den nötigen Zugang zur Vorderkammer wird ein Schnitt in die Kornea und/oder Sklera eingebracht. Zusätzlich sind zum Reduzieren eines Hornhautastigmatismus Inzisionen an der Kornea, z. B. bogenförmige Schnitte, möglich. Nachoperativ kann es im Falle eines sog. "Nachstars" erforderlich sein, den rückseitigen Kapselsack ganz oder teilweise zu entfernen. Für das Einbringen von Schnitten in den Kapselsack (auf dessen Vorder- und/oder Rückseite) wird hier der Begriff "Kapsulotomie" verwendet. Hierbei werden Kurzpuls-Laser eingesetzt, um Augengewebe z.B. mittels Photodisruption zu "schneiden".

Um mit Laserstrahlung innerhalb eines Gewebes einen Bearbeitungseffekt zu erzielen, muss die Laserstrahlung ihre Wechselwirkung auch im inneren des Gewebes entfalten. In der Augenheilkunde verwendet man deshalb Laserstrahlung, für die das Gewebe eigentlich transparent ist, und erzeugt in einem räumlich stark begrenzten Fokus eine Energiedichte, die einen Bearbeitungseffekt auslöst. Oftmals entsteht dabei ein optischer Durchbruch, der in der Literatur auch als Photodisruption bezeichnet wird. Üblicherweise ist Kurzpulslaserstrahlung im Einsatz, die auf einen engen Fokus gebündelt wird. Die Fokusgröße ist ein wesentlicher Parameter. Dies gilt auch für die Tiefenlage und Tiefenausdehnung des Fokus. Beide Parameter sind bei herkömmlichen Ablationstechniken ohne Relevanz, da diese an der Oberfläche des Materials schichtweise abtragen, also nicht innerhalb des Materials wirken.

In der US 6325792 B1 wird vorgeschlagen, Pulse eines Femtosekunden-Lasers in die Augenlinse zu fokussieren, um den Kapselsack aufzuschneiden. In der US 5246435 ist es offenbart, Pulse eines Kurzpuls-Lasers in einem dreidimensionalen Schnittmuster in die natürliche Linse des Auges zu fokussieren, um durch die Schnitte und die anschließende Blasenbildung die Linse in Bruchstücke zu fragmentieren und dadurch zu verflüssigen.

Zum Ablenken der Femtosekunden-Pulse werden zum einen feststehende Objektive und schnelle Spiegel-Scanner zur lateralen x/y-Ablenkung des Laserstrahles im Auge und langsam verstellbare Linsen zur z-Ablenkung der Fokusposition entlang einer optischen Achse des Auges eingesetzt. Solche Systeme werden etwa in der US 2006/195076 A1 oder der US 2009/131921 A1 beschrieben. Zum anderen sind auch Systeme bekannt, bei denen das Objektiv lateral langsam bewegt wird, wobei eine schnell bewegte Linse zur z-Ablenkung des Fokus entlang der optischen Achse des Auges verwendet wird. Ein solches System führt einen sog. Objektiv-Scan aus und ist z. B. in der DE 102011085046 A1 beschrieben.

Bei der Therapie des Auges durch fokussierte Pulse eines Femtosekunden-Lasers, muss der Fokus über einen gewissen Tiefenverstellbereich scharf abgebildet werden. Bei Anwendungen an der Kornea des Auges müssen zwischen 0,5 und 2 mm Fokushub realisiert werden. Bei der Anwendung sowohl an der Augenlinse als auch der Korea ist ein Fokushub von bis zu 15 mm erforderlich.

Für viele optische Anwendungen ist ein Betrieb bei zwei verschiedenen Wellenlängen wünschenswert. Solche Systeme sind z. B. aus der EP1792593 bekannt. Für die Bearbeitung von Gewebe durch nichtlineare Wechselwirkung mit Therapiestrahlung oder die Augenbeobachtung sind in der Augentherapie Anwendungen mit Femtosekunden-Pulsen entweder im Infraroten oder im Ultravioletten bekannt. Um die Energiedichte für die nichtlineare Wechselwirkung oder eine hohe Beobachtungsgüte zu erreichen, müssen die primären Farbfehler (Farblängs- und Farbquerfehler) entsprechend korrigiert sein. Aufgrund der Kurzpulseigenschaften der Strahlung ist die Korrektur nicht nur für die Schwerpunktwellenlänge erforderlich, sondern auch für den Wellenlängenbereich, der sich aufgrund der Kurzpulseigenschaft ergibt. Die Ränder des Wellenlängenbereichs stellen Nebenwellenlängen dar, für die immer noch eine gute Korrektur der Farbfehler nötig ist. Falls die Farbfehler nicht beseitigt sind, kommt es beim Durchlaufen der Optik zu einer Verbreiterung der fs-Pulse, und die für die Applikation benötigte Leistungsdichte im Fokusvolumen wird nicht erreicht. Bei Anwendungen mit UV-Strahlung beträgt z.B. die Schwerpunktwellenlänge 405 nm und der Wellenlängenbereich geht von 400 nm bis 410 nm. Die klassische Achromatisierung eines Systems bei zwei Wellenlängen - also beispielsweise bei den Nebenwellenlängen 400 nm und 410 nm - führt für Wellenlängen abseits dieses Spektralbereichs (also z.B. bei 1030 nm bis 1050 nm) zu starken Restfehlern. Diese Restfehler rühren von der charakteristischen Dispersion der technisch verfügbaren Gläser her. Durch den Einsatz von sog. Sondergläsern mit einer anomalen Dispersion lassen sich unter Umständen Farblängs- und Farbquerfehler für eine weitere Wellenlänge (z.B. die erste Mittenwellenlänge 1040 nm) korrigieren. Man benötigte dann eine Korrektur bei vier Wellenlängen (superapochromatische Korrektur), nämlich bei 400 nm, 410 nm, 1030 nm und 1050 nm. Dies ist theoretisch durch aufwändige Glaswahl rechnerisch möglich, aber praktisch nur mit sehr hohem technischem Aufwand umsetzbar. Hinderlich für diese klassische Lösung ist auch die Tatsache, dass sowohl Farblängs- als auch Farbquerfehler gleichzeitig für die vier Wellenlängen korrigiert werden müssen. In bestimmten Systemteilen führt der Einsatz von Sondergläsern zu einer Korrekturwirkung auf beide Fehler, d.h. das verwendete Sonderglas hat einen positiven Effekt sowohl auf Farblängs- als auch auf Farbquerfehler. Nach einem Zwischenbild oder nach einer Pupillenebene kehrt sich die Wirkung auf Farbquerfehler jedoch um, so dass bis zum nächsten Zwischenbild bzw. bis zur nächsten Pupillenebene die verwendeten Sondergläser für einen der beiden Fehler die Korrektur stören. Dies führt zu weiteren Aufwänden, wenn die Korrekturwirkung in den erstgenannten Systemteilen bereits ausgeschöpft ist. Eine alternative Lösung des Problems wäre der Austausch des gesamten optischen Systems (Schwenkoptik), falls der Betrieb bei den unterschiedlichen Wellenlängenbereichen nicht gleichzeitig erfolgen kann. Dies ist bedingt einen hohen Aufwand an Mechanik und Optik.

Insbesondere bei scannenden Systemen, welche den Fokus innerhalb eines Bildfeldes lateral verschieben, sind Farbquerfehler besonders störend, da sie von der Bildhöhe abhängen, die bei einem scannenden System durch die laterale Auslenkung des Scanners bedingt ist.

Der Erfindung liegt deshalb die Aufgabe zugrunde, ein System der eingangs genannten Art so auszugestalten, dass bei zwei vergleichsweise weit beabstandeten Schwerpunktwellenlängen eine Bearbeitung durch nichtlineare Wechselwirkung innerhalb eines Gewebes möglich ist.

Die Erfindung ist im Anspruch 1 definiert.

Das System ist zur Augentherapie ausgebildet, wobei Gewebe durch nichtlineare Wechselwirkung mit Therapiestrahlung bearbeitet wird. Eine Lasereinrichtung stellt Therapiestrahlung bereit. Eine Fokussiereinrichtung hat ein in einem Therapievolumen gelegenes Bildfeld und bündelt die Therapiestrahlung zu einem Fokus im Bildfeld. Der Fokus und die Lasereinrichtung sind so ausgestaltet, dass innerhalb des Gewebes die Bearbeitung durch nichtlineare Wechselwirkung erfolgt. Der Fokussiereinrichtung ist eine xy-Scannereinrichtung vorgeordnet. Sie verschiebt den Fokus lateral im Bildfeld. Weiter weist das System eine z-Scannereinrichtung auf, die eine Tiefenlage des Fokus im Therapievolumen verstellt. xy-Scanner und z-Scanner sind von einer Steuereinrichtung angesteuert. Die Lasereinrichtung stellt die Therapiestrahlung bereit. Eine erste Kurzpulsstrahlung liegt bei einer ersten Schwerpunktwellenlänge. Eine zweite Kurzpulsstrahlung liegt bei einer zweiten Schwerpunktwellenlänge, die von der ersten Schwerpunktwellenlänge um mindestens 300 nm, bevorzugt 500 nm, beabstandet ist. Die beiden Kurzpulsstrahlungen decken aufgrund der Pulslänge, insbesondere im Pulslängenbereich zwischen 1 ps und 1 fs, einen Wellenlängenbereich ab, die jeweilige Schwerpunktwellenlänge einschließt. Das System hat mindestens zwei Betriebsmodi. In einem ersten Betriebsmodus arbeitet es mit der ersten Kurzpulsstrahlung, in einem zweiten Betriebsmodus mit der zweiten Kurzpulsstrahlung. Es ist hinsichtlich Farblängsfehler und Farbquerfehler derart ausgebildet, dass das System bei mindestens einer der beiden Schwerpunktwellenlängen einen Farblängsfehler hat. Eine spektrale Kennlinie des Farblängsfehlers im ersten und im zweiten Wellenlängenbereich verläuft im Wesentlichen horizontal. Sie ist im Rahmen einer Farblängsfehlertoleranz konstant. Das System ist hinsichtlich Farbquerfehler weiter dahingehend ausgebildet, dass die Fokussiereinrichtung bei mindestens einer der beiden Schwerpunktwellenlängen einen Farbquerfehler zeigt. Eine spektrale Kennlinie des Farbquerfehlers ist in beiden Wellenlängenbereichen im Rahmen einer Farbquerfehlertoleranz konstant. Die Kennlinie zeigt also in den Wellenlängenbereichen im Wesentlichen einen horizontalen Verlauf. Die Steuereinrichtung ist ausgebildet, die xy-Scannereinrichtung so anzusteuern, dass der Farbquerfehler durch eine entsprechende Ablenkfunktion für die laterale Verschiebung des Fokus ausgeglichen ist.

Es kann aber auch für die Untersuchung ausgebildet sein. Die Therapiestrahlung ist dann Untersuchungsstrahlung bzw. Messstrahlung, das Therapievolumen ein Untersuchungsvolumen. Die Untersuchung kann dabei mittels nichtlinearer Wechselwirkung, beispielsweise Multi-Photonenprozessen erfolgen. Dies aber nicht zwingend. Entscheidend ist für eine derartige Modifikation des Systems lediglich, dass durch die Kurzpulsnatur der Strahlung, insbesondere im Pulslängenbereich zwischen 1 ps und 1 fs, sich Wellenlängenbereiche für die erste bzw. zweite Kurzpulsstrahlung ergeben, innerhalb denen die Farblängsfehler und Farbquerfehler im Rahmen der geschilderten Toleranzen konstant sind.

Im System zur Augentherapie durch Bearbeitung von Gewebe mittels nichtlinearer Wechselwirkung mit Therapiestrahlung ist die Lasereinrichtung vorgesehen, welche die Therapiestrahlung bereitstellt. Die Therapiestrahlung wird von der Fokussiereinrichtung im Bildfeld gebündelt, und xy-Scanner sowie z-Scanner verschieben den Fokus lateral und in der Tiefe in einem Therapievolumen. Die Therapiestrahlung hat durch die Kurzpulseigenschaften Wellenlängenbereiche. Das System ist hinsichtlich Farblängsfehler und Farbquerfehler besonders korrigiert, so dass die spektralen Kennlinien beider Fehler in den Wellenlängenbereichen jeweils ein lokales Extremum haben, wobei in den Wellenlängenbereichen eine bestimmte Toleranz nicht überschritten wird, die Kennlinien also sehr flach sind.

Der Farblängsfehler bzw. dessen spektraler Kennlinie beschreibt die axiale Variation der Fokusposition in Abhängigkeit von der Wellenlänge. Der Farblängsfehler wird bezogen auf die axiale Fokusposition einer Bezugswellenlänge angegeben, beispielsweise - aber nicht zwingend - bezogen auf die erste oder die zweite Schwerpunktwellenlänge. Der Farblängsfehler der Bezugswellenlänge ist dann per Definition gleich Null; die Kennlinie hat einen Nullpunkt.

Das System hat bei mindestens einer der Schwerpunktwellenlängen einen Farblängsfehler. Nimmt man eine der beiden Schwerpunktwellenlängen als Bezugswellenlänge für den Farblängsfehler, liegt dieser bei der anderen der beiden Schwerpunktwellenlängen vor. Ist keine der beiden Schwerpunktwellenlängen die Bezugswellenlänge für den Farblängsfehler, hat das System bei beiden Schwerpunktwellenlängen einen Farblängsfehler. Die Konstanz der Kennlinie innerhalb der Farblängsfehlertoleranz hat zur Folge, dass das System innerhalb des ersten Wellenlängenbereiches keinen Farblängsfehler hat, wenn man eine Wellenlänge aus diesem Wellenlängenbereich als Bezugswellenlänge wählt, z. B. die erste Schwerpunktwellenlänge. Gleiches gilt für den zweiten Wellenlängenbereich, wenn man eine Wellenlänge aus diesem Wellenlängenbereich als Bezugswellenlänge wählt.

Analoges gilt für den Farbquerfehler. Der Farbquerfehler bzw. dessen spektraler Kennlinie beschreibt die laterale Variation der Fokusposition quer zur optischen Achse bzw. zur Lichtausbreitungsrichtung in Abhängigkeit von der Wellenlänge. Der Farbquerfehler wird bezogen auf die laterale Fokusposition einer Bezugswellenlänge angegeben, beispielsweise - aber nicht zwingend - bezogen auf die erste oder die zweite Schwerpunktwellenlänge. Der Farblängsfehler der Bezugswellenlänge ist dann per Definition gleich Null; die Kennlinie hat einen Nullpunkt.

Das System hat bei mindestens einer der Schwerpunktwellenlängen einen Farbquerfehler. Nimmt man eine der beiden Schwerpunktwellenlängen als Bezugswellenlänge für den Farbquerfehler, liegt dieser bei der anderen der beiden Schwerpunktwellenlängen vor. Ist keine der beiden Schwerpunktwellenlängen die Bezugswellenlänge für den Farbquerfehler, hat das System bei beiden Schwerpunktwellenlängen einen Farbquerfehler. Die Konstanz der Kennlinie innerhalb der Farbquerfehlertoleranz hat zur Folge, dass das System innerhalb des ersten Wellenlängenbereiches keinen Farbquerfehler hat, wenn man eine Wellenlänge aus diesem Wellenlängenbereich als Bezugswellenlänge wählt, z. B. die erste Schwerpunktwellenlänge. Gleiches gilt für den zweiten Wellenlängenbereich, wenn man eine Wellenlänge aus diesem Wellenlängenbereich als Bezugswellenlänge wählt.

Die Fehlertoleranzen lassen es zu, dass die entsprechenden Farblängsfehler bzw. Farbquerfehler innerhalb der genannten Wellenlängenbereiche noch eine geringfügige Schwankung haben können, im Falle des Farblängsfehlers ist das vom Fokus der Kurzpulsstrahlung abgedeckte Volumen also axial aufgeweitet, im Fall des Farbquerfehlers lateral aufgeweitet. Der Begriff "geringfügig" ist daran bemessen, dass die Aufweitung die Bedingungen für die Wirkung der Kurzpulsstrahlung, also zum Erzeugen der nichtlinearen Wechselwirkung oder der gewünschten Beobachtungsgüte, nicht zerstört. Mit anderen Worten, die Farblängsfehlertoleranz ist so bemessen, dass für die betroffene Kurzpulsstrahlung die axiale Aufweitung des Fokusvolumens auf ein Ausmaß begrenzt bleibt, dass die Erzeugen der nichtlinearen Wechselwirkung nicht oder nicht unzulässig beeinträchtigt wird. Gleiches gilt in lateraler Richtung für den Farbquerfehler. In Ausführungsformen beträgt die Farblängsfehlertoleranz z. B. ein bestimmtes Vielfaches der Schärfentiefe, beispielsweise 0,1 x, 0,2 x, 0,5 x, 1 x oder 2 x der Schärfentiefe der Fokussiereinrichtung. In anderen, damit kombinierbaren Ausführungsbeispielen beträgt die Farbquerfehlertoleranz ein bestimmtes Vielfaches der lateralen Fokusgröße, bezogen auf den sogenannten Airy-Durchmesser, beispielsweise 0,1 x, 0,2 x, 0,5 x, 1 x oder 2 x des Airy-Durchmessers. Diese Konstanz der Kennlinie ist deshalb gefordert, da aufgrund der Kurzpulseigenschaft die Kurzpulsstrahlungen den Wellenlängenbereich um die jeweilige Schwerpunktwellenlänge herum abdecken. Das kann nicht verhindert werden. Im Falle einer Schwerpunktwellenlänge von 405 nm ist der Wellenlängenbereich beispielsweise 10 nm breit. Bei einer Schwerpunktwellenlänge von 1040 nm ist er beispielsweise 20 nm breit. Die Konstanz von Farbquerfehler und Farblängsfehler innerhalb der entsprechenden Toleranz stellt sicher, dass die Wellenlängenbereiche im System und insbesondere durch die Fokussiereinrichtung so geführt werden, dass ihre Breite zu keiner Aufweitung des Fokusvolumen führt, die so groß wäre, dass keine nichtlineare Wechselwirkung mehr erreicht bzw. diese nicht mehr in ausreichendem Umfang erreicht wird oder die Beobachtungsgüte verfehlt würde.

Durch die Anforderung an die spektralen Kennlinien kann das System auf eine Apochromatisierung der Optiken verzichten. Der verbleibende (wenn auch konstante) Farbquerfehler bei mind. einer der Schwerpunktwellenlängen führt dazu, dass ein an der xy-Scannereinrichtung angesteuerter Ablenkwinkel durch den Farbquerfehler eine zusätzliche oder verminderte laterale Ablenkung im Therapievolumen erfährt. Da der Farbquerfehler bekannt ist, sorgt die Steuereinrichtung durch eine entsprechende Ansteuerung der xy-Scannereinrichtung dafür, dass diese laterale Verschiebung des Fokus ausgeglichen ist. Eine aufwändige Korrektur wird also überraschend einfach durch eine entsprechende Ansteuerung der xy-Scannereinrichtung ersetzt.

Der Farblängsfehler, der zwischen der zweiten und der ersten Schwerpunktwellenlänge besteht kann ebenfalls durch die Steuereinrichtung ausgeglichen werden, indem diese die z-Scannereinrichtung so ansteuert, dass bei Betrieb mit der vom Farblängsfehler betroffenen Kurzpulsstrahlung durch einen entsprechenden Offset der Tiefenlage des Fokus ausgeglichen ist. Aufgrund der geforderten Konstanz der spektralen Kennlinie des Farblängsfehlers äußert sich der Farblängsfehler ja ausschließlich durch einen Offset der Tiefenlage des Fokus, ohne über den ersten Wellenlängenbereich eine unzulässige axiale Fokusverformung zu bewirken.

Alternativ oder zusätzlich durch den entsprechenden Ausgleich des Farblängsfehlers durch die z-Scannereinrichtung ist es möglich, ein nur auf die betroffene Kurzpulsstrahlung wirkendes oder nur dann aktiviertes Umfokussierungselement zu verwenden, das den Farblängsfehler durch einen entsprechenden Offset der Tiefenlage des Fokus teilweise ausgleicht oder gänzlich ausgleicht.

Den wechselweisen Betrieb des Systems mit den beiden Kurzpulsstrahlungen, d.h. die eingangs genannten Betriebsmodi, kann man aufwandsgering in Ausführungsformen dadurch realisieren, dass das System einen ersten Eingangsstrahlengang für die erste Kurzpulsstrahlung und einen zweiten Eingangsstrahlungsgang für die zweite Kurzpulsstrahlung aufweist und weiter eine der Fokussierungseinrichtung vorgeordnete Vereinigungseinrichtung hat, welche die beiden Eingangsstrahlengänge zusammenführt oder zwischen diesen beiden umschaltet. Auf diese Weise ist es möglich, die Korrekturen für den Farblängsfehler in den jeweiligen Eingangsstrahlengängen vorzusehen oder, was bevorzugt ist, den ersten Eingangsstrahlengang oder die Fokussierungseinrichtung derart zu korrigieren, dass die spektrale Kennlinie des Farblängsfehlers in diesem Wellenlängenbereich im Rahmen der genannten Farblängsfehlertoleranz konstant ist. Dann ist in Ausführungsformen im Strahlengang nach der Vereinigungseinrichtung ein von der Steuereinrichtung angesteuertes Korrekturglied angeordnet, das hinsichtlich einer Korrekturwirkung für den zweiten Wellenlängenbereich aktivierbar ist. Bei aktiver Korrekturwirkung modifiziert es die Optiken so, dass die im Rahmen der Farblängsfehlertoleranz konstante, spektrale Kennlinie des Farblängsfehlers im zweiten Wellenlängenbereich gegeben ist. Die Steuereinrichtung aktiviert das Korrekturglied hinsichtlich dessen Korrekturwirkung, nur wenn die zweite Kurzpulsstrahlung durch das System läuft. Es deaktiviert das Korrekturglied hinsichtlich seiner Korrekturwirkung, wenn die erste Kurzpulsstrahlung durch das System läuft, da das System hierfür ja schon korrigiert ist. Eine Optik, die für die erste Kurzpulsstrahlung bereits optimal farbkorrigiert ist, kann dadurch so aufwandsgering für den Betrieb mit der zweiten Kurzpulsstrahlung erweitert werden, nämlich durch das genannte Korrekturglied. Um möglichst keinen Farbquerfehler zu erzeugen, ist es zu bevorzugen, das Korrekturglied in einer Pupille der Fokussierungsoptik anzuordnen, bevorzugt dort einzuschwenken. Es kann auch als verstellbares Optikelement ausgebildet sein, insbesondere als Variolinse oder MEMS-Array, das von der Steuereinrichtung hinsichtlich seiner Korrekturwirkung aktiviert bzw. deaktiviert wird, indem das Optikelement entsprechend eingestellt wird.

Alternativ zu einem Korrekturglied, das nach der Vereinigereinrichtung vorsehen ist, kann auch die Vereinigereinrichtung so ausgebildet werden, dass sie ein Korrekturglied der genannten Eigenschaften enthält, welches nur auf die zweite Kurzpulsstrahlung wirkt.

Besonders bevorzugt ist aufgrund ihrer baulichen Einfachheit eine Ausführungsform mit den beiden Eingangsstrahlengängen, bei der der erste Eingangsstrahlengang oder die Fokussiereinrichtung die Korrektur hinsichtlich der konstanten spektralen Kennlinie des Farblängsfehlers im ersten Wellenlängenbereich aufweist und das Korrekturglied, welches die Kennlinienanpassung des Farblängsfehlers für den zweiten Wellenlängenbereich bewirkt, im zweiten Eingangsstrahlengang vorgesehen ist. Diese Ausführungsform erlaubt es insbesondere die z-Scannereinrichtung einteilig auszubilden, mit einem zweiten z-Scanner für die zweite Kurzpulsstrahlung im zweiten Eingangsstrahlengang und einem ersten z-Scanner für die erste Kurzpulsstrahlung im ersten Eingangsstrahlengang. Man kann dann das Korrekturglied direkt im zweiten z-Scanner anordnen, insbesondere in einem festen Optikglied eines Teleskops, das den zweiten z-Scanner in Form eines beweglichen und eines festen Optikgliedes realisiert.

In einer Ausführungsform ist die Funktion des Korrekturgliedes in dem bereits genannten Umfokussierungselement ausgebildet.

In bevorzugten Ausführungsformen ist die Fokussiereinrichtung für einen der beiden Wellenlängenbereiche auch hinsichtlich monochromatischer Fehler korrigiert. Im anderen Wellenlängenbereich hat sie einen monochromatischen Restfehler und in einem nur von der Strahlung dieses Wellenlängenbereichs durchstrahlten Strahlengang befindet sich eine Ausgleichsoptik, die den monochromatischen Restfehler ausgleicht. Diese Bauweise ist besonders vorteilhaft, da wiederum ein bereits für eine Kurzpulsstrahlung ausgebildetes Optikdesign aufwandsgering für eine andere Kurzpulsstrahlung erweitert werden kann.

Ausgleichsoptik und Korrekturglied und optional auch Umfokussierungselement können in bevorzugten Ausführungsformen in einem Korrekturoptikglied zusammengefasst sein.

Die Schwerpunktwellenlänge liegt bevorzugt nicht über 420 nm, weiter bevorzugt zwischen 380 und 420 nm, besonders bevorzugt bei 405 nm. Die andere Schwerpunktwellenlänge liegt bevorzugt nicht unter 730 nm, weiter bevorzugt zwischen 1030 und 1060 nm, besonders bevorzugt bei 1040 nm. Jede der beiden kann die erste oder zweite Schwerpunktwellenlänge sein. Die beiden Wellenlängenbereiche sind bevorzugt nicht breiter als 30 nm, besonders bevorzugt nicht breiter als 15 nm.

Für Anwendungen in der Korneachirurgie beträgt der z-Hub der z-Scannereinrichtung bevorzugt mindestens 1 mm für Anwendungen an der flachgedrückten Kornea, weiter bevorzugt mindestens 3 mm für Anwendungen an der gekrümmten Kornea, besonders bevorzugt mindestens 5 mm für Arbeiten an der Augenlinse und weiter besonders bevorzugt mindestens 13 mm für Anwendungen in der Katarakt-OP.

Da die z-Scannereinrichtung ein mechanisch bewegtes Bauteil umfasst, das einen vergleichsweise großen Hub ausführt, ist es für ophthalmologische Anwendungen bevorzugt, solche Bauteile möglichst fern vom Patienten anzuordnen. Dadurch werden Schwingungen und Geräusche, die den Patienten irritieren könnten, vermieden. Es ist deshalb bevorzugt, dass die z-Scannereinrichtung mind. ein dem xy-Scanner vorgeordnetes, divergenzvariierendes optisches Element umfasst, das die Divergenz der Therapiestrahlung einstellbar verändert. Besonders bevorzugt ist hierbei eine Ausgestaltung mit einem Teleskop mit einer feststehenden Sammellinsenoptik und einer beweglichen Linsenoptik. Bei Ausführungsformen mit zwei z-Scannern kann ein oder können beide z-Scanner so ausgebildet sein. Das Teleskop wird man zweckmäßigerweise als Galilei-Teleskop ausführen.

Scannende Systeme bestehen zum einen aus optischen Komponenten, die sich in Lichtrichtung vor den scannenden Elementen befinden. Diese Komponenten werden nur axial genutzt. Nach den scannenden Elementen befinden sich zum anderen optische Komponenten (Fokussierungseinrichtung; auch als Scanoptik bezeichnet), die auch außeraxiale Feldbündel zum Bild hin abbilden.

Beispiele für optische Komponenten vor den Scannern sind unter anderem Kollimatoren, Strahlaufweiter, polarisationsoptische Elemente oder Systeme zur Divergenzvariation, d.h. zur Fokussierung.

Abbildungsfehler, die feldunabhängig sind, d.h. die sowohl auf der optischen Achse als auch abseits der optischen Achse im selben Maß auftreten, lassen sich ggf. bereits durch Elemente vor den Scannern korrigieren. Hierzu zählen insbesondere Farblängsfehler und sphärische Aberration.

In Ausführungsformen wird die Korrektur des Systems für die zwei Wellenlängenbereiche wie folgt durchgeführt:
(i) Die Scanoptik wird zunächst für einen der beiden Spektralbereiche (hier 400 nm bis 410 nm) mit klassischen Mitteln nach Stand der Technik achromatisiert und für die Mittenwellenlänge für die sonstigen Fehler korrigiert.
(ii) Für die beiden Wellenlängenbereiche existieren weiter unterschiedliche Optiken vor den scannenden Elementen. Diese optischen Systeme werden i.d.R. ohnehin in zwei Ausführungen benötigt, da die beiden Lichtquellen oftmals unterschiedliche Anforderungen an die Brennweite der Kollimatoren stellen, oder weil für die unterschiedlichen Bereiche unterschiedliche Divergenzvariationen nötig sind. Vor dem xy-Scanner können die beiden optischen Systeme beispielsweise über einen dichroitischen Strahlteiler verlustarm kombiniert werden. Wegen der ausschließlich axialen Nutzung sind diese optischen Systeme i.d.R. von einfacher Gestalt.
(iii) Die Scanoptik nach dem xy-scannenden Element wird durch Einsatz von Sondergläsern nur teilweise korrigiert. Die passenden Sondergläser werden hauptsächlich in Systemteilen eingesetzt, die sowohl auf Farblängs- als auch auf Farbquerfehler korrigierend wirken. Es wird der Farbquerfehler hierbei für sich genommen so beeinflusst, dass im betrachteten ersten Wellenlängenbereich geringstmögliche Änderungen verbleiben. Im Farbquerfehlerdiagramm äußert sich dies durch eine Kurve mit verschwindender Steigung. Eine zweite Nullstelle (Apochromasie), d.h. eine vollständige Korrektur einer zweiten Wellenlänge im klassischen Sinne, ist nicht zwingend erforderlich. Dadurch wird der Aufwand an Sondergläsern im Vergleich zur klassischen Lösung reduziert. Beim Betrieb im anderen Spektralbereich äußert sich der verbleibende Offset zwischen den beiden Wellenlängenbereichen als Brennweitenänderung bzw. Abbildungsmaßstabsänderung. Diese kann durch eine angepasste Optik vor dem xy-Scanner (Anpassung des Eingangsstrahldurchmessers) und durch eine Anpassung der Kippwinkel des xy-Scanners kompensiert werden und ist applikativ nicht von Belang.
(iv) Die Farblängsfehlerkurve ist dann im Allgemeinen nicht korrigiert. Sie weist vielmehr einen Offset zum zweiten Wellenlängenbereich auf (=Fokusablage) und eine nicht-verschwindende Steigung auf (=primärer Rest-Farblängsfehler zwischen 1030 nm und 1050 nm). Der Offset kann durch Umfokussieren korrigiert werden. Dies geschieht erfindungsgemäß durch eine angepasste Divergenz in der Optik vor dem xy-Scanner. Der verbleibende primäre Farblängsfehler ist feldunabhängig und kann folglich durch ein Korrekturglied ebenfalls vor dem xy-Scanner, das sich nur im Strahlengang für den zweiten Wellenlängenbereich befindet, kompensiert werden. Der Betrieb im ersten Wellenlängenbereich wird dadurch nicht behindert, da sich das Element dort nicht im Strahlengang befindet.
   Beispiele für ein solches Korrekturglied sind unter anderem Kittglieder (Kombinationen aus Positiv- und Negativlinsen mit unterschiedlichen Glassorten) oder diffraktive Elemente.
(v) Nach Korrektur der Farbfehler für beiden Wellenlängenbereiche ist noch die Korrektur der monochromatischen Fehler nötig. Im ersten Wellenlängenbereich geschah dies laut (i) bereits mit klassischen Mitteln, die aus dem Stand der Technik bekannt sind, d.h. durch eine geeignete Wahl der Linsenradien der Scanoptik. Im zweiten Wellenlängenbereich verbleiben dann aber Restfehler. Die verbleibende sphärische Aberration (=feldunabhängig) kann ebenfalls durch ein Korrekturelement vor dem xy-Scanner erfolgen. Dies kann beispielsweise durch eine asphärische Fläche auf dem Kittglied nach (iv) erfolgen, oder durch weitere sphärische Linsen mit geeigneter Gestalt, oder durch passende diffraktive Elemente mit asphärischer Wirkung.
(vi) Die Korrektur der feldabhängigen monochromatischen Fehler kann nur noch in der Optik nach dem xy-Scanner erfolgen. Dies kann durch Vermittelung beider Spektralbereiche erfolgen, d.h. durch eine Lösung, die tatsächlich für diese Fehler korrigiert ist - oder deren Restfehler für die Applikation noch akzeptiert werden kann. Auch hierfür können ggf. asphärische Flächen in der Scanoptik eingesetzt werden. Formal handelt es sich um eine Korrektur von Abbildungsfehlern bei zwei Wellenlängen (= den beiden Mittenwellenlängen), d.h. eine klassische und i.d.R. weniger aufwändige Achromatisierung. Wegen der bereits kompensierten primären Farbfehler für beide Nebenwellenlängen ist der technische Aufwand somit moderat. In Fällen, in denen diese Lösung noch nicht zufriedenstellend ist, können ggf. Teile der Scanoptik für den Betrieb in beiden Spektralbereichen ausgewechselt werden - dann verschwindet aber die Möglichkeit der zeitgleichen Nutzung. Dies kann durch ein automatisiertes Wechselsystem erfolgen.
(vii) Die Korrekturglieder in der Optik vor dem xy-Scanner müssen in einer zu den scannenden Elementen kongruierten optischen Ebene, d.h. in einer Pupillenebene angebracht werden. Dies ist insbesondere der Fall, wenn das System durch Divergenzvariation vor dem xy-Scanner in unterschiedlichen Fokustiefen betrieben wird. Falls sich die Korrekturglieder nicht in einer Pupillenebene befinden, so ändert sich der genutzte freie Durchmesser der Elemente bei verschiedenen Fokusstellungen, und die Korrekturwirkung der Elemente wird fokusabhängig gestört. Erfindungsgemäß wird dies durch eine 4-f-Relayoptik gelöst, die zwischen dem divergenzvariierenden Element und dem xy-Scanner eingefügt wird. Das Korrekturglied befindet sich dann im vorderen Brennpunkt der zweiten Gruppe des 4-f-Systems, und der xy-Scanner befindet sich im hinteren Brennpunkt der ersten Gruppe des 4-f-Systems. Die beiden Gruppen haben ungefähr den Abstand der Summe der beiden Teilbrennweiten der Gruppen (=4-f-System). In der zum xy-Scanner kongruenten Ebene ist dann mechanischer Raum zum Einfügen der Korrekturelemente vorhanden. Falls mehrere Elemente eingebracht werden müssen, kann dieses Relay auch mehrfach hintereinander zum Einsatz kommen. Eine Ausführung als Spiegeloptik (z. Bsp. Offner-System) ist ebenfalls denkbar.

Nachfolgend wird die Erfindung beispielsweise anhand der beigefügten Zeichnungen, die auch erfindungswesentliche Merkmale offenbaren, noch näher erläutert. Es zeigen:
Fig. 1 eine Schemadarstellung einer Optik eines Gerätes zur lasergestützten Augenchirurgie, das bei zwei Wellenlängen arbeiten kann, wobei der Strahlengang schematisch dargestellt ist,
Fig. 2 einen z-Scanner, der im Gerät der Fig. 1 zur Anwendung kommt,
Fig. 3 einen Strahlvereiniger und xy-Scanner, der im Gerät der Fig. 1 zur Anwendung kommen kann,
Fig. 4 ein Korrekturglied, das für eine Wellenlänge im Gerät der Fig. 1 verwendet wird,
Fig. 5 eine Kennlinie des Farblängsfehlers des Gerätes der Fig. 1,
Fig. 6 eine Kennlinie des Farbquerfehlers des Gerätes der Fig. 1,
Fig. 7 eine vergrößerte Darstellung der Fokussierung der Therapiestrahlung in eine Augenhornhaut und
Fig. 8 eine Darstellung ähnlich der Fig. 5 für eine weitere Ausführungsform des Gerätes der Fig. 1, die sich hinsichtlich der Korrektur des Farbquerfehlers unterscheidet.

Die Erfindung wird nachfolgend mit Bezug auf die Augenchirurgie beschrieben, was lediglich exemplarisch für verschiedene Aufgaben der Augentherapie stehen soll, für welche die verschiedenen Aspekte der Erfindung einsetzbar sind.

In den folgenden Beispielen wird eine lasergestützte Augenchirurgie beschrieben, die fs-Laser nutzt, die die am häufigsten im Bereich der lasergestützten Augenchirurgie genutzten Kurzpuls-Laser sind - und damit auch die am besten untersuchten. Dennoch sind alle hier beschriebenen Systeme auch mit anderen Kurzpuls-Lasern umsetzbar. fs-Laser stehen also, sofern nicht explizit auf die Pulslänge als differenzierendes Merkmal eingegangen wird, als Synonym für Kurzpulslaser.

Das hier beschriebene System, an dem die verschiedenen Aspekte der Erfindung rein exemplarisch in Kombination realisiert sind, dient zur lasergestützten Kornea-Operation. Mittels der Kurzpuls-Laserstrahlquelle werden Schnitte in der Hornhaut ausgeführt, beispielsweise zur Fehlsichtigkeitskorrektur. Das System kann aber auch zur Katarakt-OP ausgestaltet sein, z.B. um einen Zugangsschnitt zur Vorderkammer des Auges durch die Kornea, einen Kapsulotomie-Schnitt, Schnitte zum Zerkleinern des Linsenkerns des Auges auszuführen.

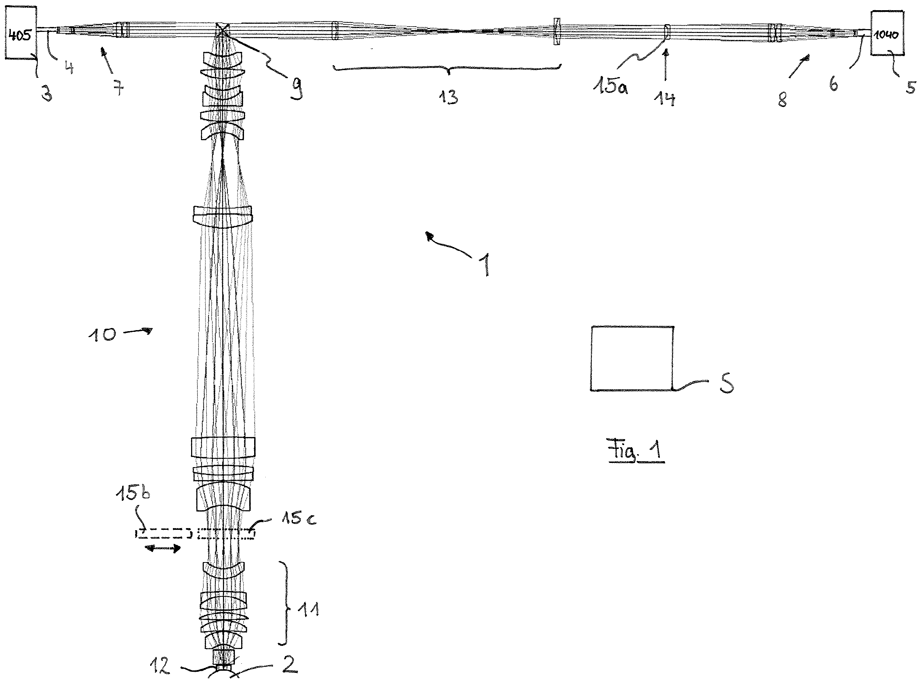

Fig. 1 zeigt schematisch den Strahlengang eines Therapiesystems 1 für die Augenheilkunde, insbesondere für die Kornea-Chirurgie. Durch Erzeugen von Schnittflächen in der Kornea eines Auges 2. Das Therapiesystem 1 ist ausgebildet, bei zwei Wellenlängen zu arbeiten, nämlich bei einer ersten Schwerpunktwellenlänge von 405 nm und einer zweiten Schwerpunktwellenlänge von 1040 nm. Die Wellenlängen werden einzeln genutzt, d.h. das Therapiesystem 1 ist zwischen den beiden Wellenlängen umschaltbar. In einer Ausführungsform des Therapiesystems 1 stellt eine erste Laserquelle 3 einen ersten Laserstrahl 4 bei der ersten Schwerpunktwellenlänge von 405 nm zur Verfügung, und eine zweite Laserquelle 5 stellt einen zweiten Laserstrahl 6 bei der zweiten Schwerpunktwellenlänge von 1030 nm bereit. Die Laserquellen 3, 6 sind Kurzpulslaserquellen, so dass die Laserstrahlen 4, 6 gepulste Laserstrahlung mit einer Pulslänge z.B. im fs-Bereich sind.

Für den ersten Laserstrahl 4 ist ein z-Scanner 7 vorgesehen, der zur Verstellung der Tiefenlage eines noch zu erläuternden Fokus in der Hornhaut des Auges 2 ausgebildet ist. Gleichermaßen wird der zweite Laserstrahl 6 mit einem zweiten z-Scanner 8 in der Tiefe verstellt. Eine xy-Scannereinrichtung 9 ist zugleich als vereinigendes Element für die beiden Strahlengänge des zweiten Laserstrahls 6 und des ersten Laserstrahls 4 ausgebildet und speist einen der beiden Laserstrahlen gescannt in den Strahlengang einer Fokussieroptik 10. Die Auswahl zwischen den beiden Strahlen kann dabei entweder dadurch erfolgen, dass die in der xy-Scannereinrichtung 9 vorgesehene Vereinigeroptik zwischen einem der beiden Strahlen umschaltet, so dass nur einer der beiden Strahlen gescannt durch die Fokussieroptik 10 läuft. Alternativ kann eine kontinuierliche Zusammenführung der beiden Strahlengänge erfolgen und es wird nur eine der beiden Laserquellen 3, 5 aktiviert. Dies kann von einer Steuereinrichtung S, die den gesamten Betrieb des Therapiesystems 1 steuert und dabei insbesondere auch die Laserquellen 3, 5, die xy-Scannereinrichtung 9 und die z-Scanner 7, 8 ansteuert, entsprechend eingestellt werden.

Die Fokussieroptik 10 umfasst ein Objektiv 11 und bündelt den von der xy-Scannereinrichtung 9 lateral gescannten ersten oder zweiten Laserstrahl 4, 6 durch ein Kontaktglas 12 in die Hornhaut des Auges 2.

Die beiden Schwerpunktwellenlängen sind um mehr als 500 nm beabstandet. Die Fokussierung in die Hornhaut und die Ausgestaltung der Laserquellen 3, 5 ist so gestaltet, dass die Pulse der Laserstrahlen 4, 6 einen optischen Durchbruch in der Augenhornhaut erzeugen oder über eine durchbruchsfreie, nichtlineare Wechselwirkung Gewebeschichten in der Augenhornhaut trennen. Damit dieser Effekt eintritt, muss eine große Fokusgüte erreicht werden. Der spektrale Unterschied zwischen erstem und zweitem Laserstrahl 4, 6 würde bei einer Optik, die ausschließlich für eine der beiden Wellenlängen ausgelegt ist, dazu führen, dass bei der anderen Wellenlänge chromatische Fehler aufträten. Diese hätten zur Folge, dass eine gewünschte Fokusposition nicht mehr erreicht würde. Zudem wäre für diejenige Wellenlänge, für welche die Optik des Therapiesystems 1 nicht ausgelegt wäre, gar kein optischer Durchbruch bzw. keine nichtlineare Wechselwirkung mehr zu erreichen. Aufgrund der Kurzpulseigenschaften der ersten und zweiten Laserstrahlung 4, 6 haben die Laserstrahlen um die genannten Schwerpunktwellenlängen eine gewisse Bandbreite, decken also jeweils einen Wellenlängenbereich ab. Die Breite dieser Wellenlängenbereiche ist aufgrund physikalischer Gesetze mit der Kürze der Laserpulse der Laserstrahlen untrennbare verknüpft.

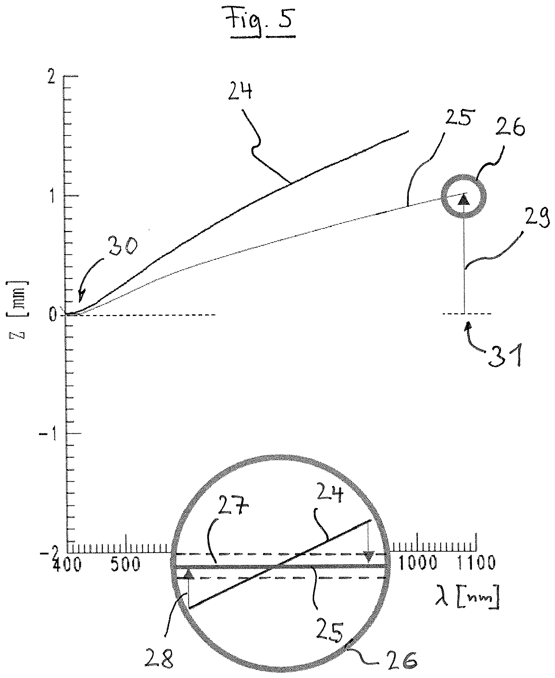

Um bei beiden Betriebsarten, d.h. bei Betrieb sowohl mit dem ersten Laserstrahl 4 als auch bei Betrieb mit dem zweiten Laserstrahl 6 zuverlässig die nichtlineare Wechselwirkung im Fokus, der von der Fokussieroptik 10 samt Objektiv 11 erzeugt wird, zu gewährleisten, ist das Therapiesystem 1 für die erste Wellenlänge, d.h. für die Strahlung des ersten Laserstrahls 4 sowie für die Strahlung des zweiten Laserstrahls 6 und den dadurch bestimmten zweiten Wellenlängenbereich auf bestimmte Weise korrigiert. Diese Korrektur wird nachfolgend anhand der Fig. 5, 6 und 8 noch näher erläutert werden.

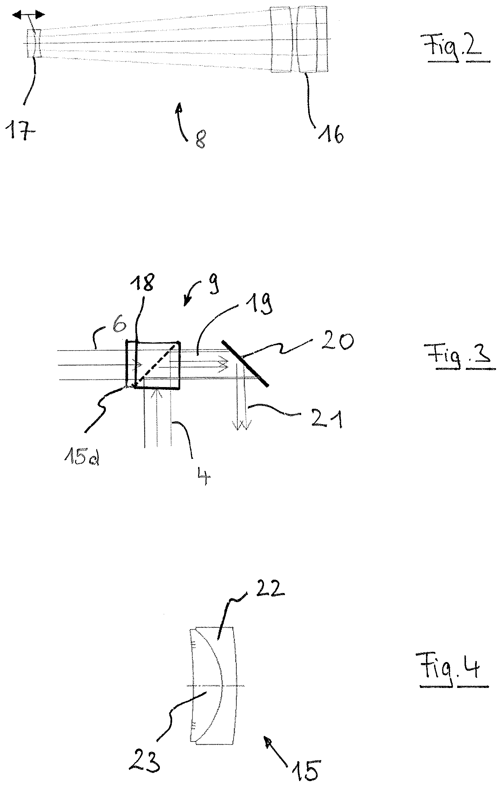

Fig. 2 zeigt exemplarisch den z-Scanner 7 oder 8. Er ist als Galilei-Teleskop mit einer feststehenden Sammeloptik 16 und einer beweglichen Zerstreuungsoptik 17 ausgebildet. Durch Verschiebung der beweglichen Zerstreuungsoptik 17 wird die Divergenz der Strahlung nach dem z-Scanner 7, 8 entsprechend eingestellt, wodurch sich letztlich zusammen mit der Fokussieroptik 10 die Tiefenlage des Fokus im Auge 2 einstellen lässt.

Fig. 3 zeigt eine schematische Ausführungsform für die xy-Scannereinrichtung 9, die einen dichroitischen Vereiniger 18 aufweist, der den zweiten Laserstrahl 6 und den ersten Laserstrahl 4 zusammenführt und als gemeinsamen Strahlengang 19 zu einem Scannerspiegel-Paar 20 leitet. Von dort liegt ein gescannter Strahlengang 21 vor, der dann durch die Fokussieroptik 10 und das Objektiv 11 sowie das Kontaktglas 12 läuft.

In der Darstellung der Fig. 1 befindet sich nach dem zweiten z-Scanner 8 eine Relayoptik 13, so dass im Strahlengang zwischen dem z-Scanner 8 und der xy-Scannereinrichtung 9 eine Pupillenebene 14 geschaffen ist. In dieser liegt ein Korrekturoptikglied 15a, das eine bestimmte Funktion hinsichtlich der Farbkorrektur des Therapiesystems 1 hat, die nachfolgend anhand der Fig. 5, 6 und 8 deutlich werden wird.

In Fig. 3 ist weiter ein Korrekturoptikglied 15d eingezeichnet, das am dichroitischen Vereiniger 12 vorgesehen sein kann und auf den zweiten Laserstrahl 6 wirkt. Dieses Korrekturoptikglied 15d ist eine Alternative zur Verwendung des Korrekturoptikglieds 15a. Auch seine Funktion wird noch erläutert werden.

Fig. 5 zeigt den spektralen Verlauf des Farblängsfehlers Z des Therapiesystems 1 in Therapievolumen, d.h. in dem Volumen, in dem das Auge 2 zu liegen kommt. Die Kennlinie 24 zeigt den Farblängsfehlerverlauf über der Wellenlänge λ für ein herkömmliches Gerät, das also nur für eine Schwerpunktwellenlänge ausgelegt ist. Wie zu sehen ist, steigt der Farblängsfehler Z von einer Wellenlänge von 400 nm an. Zwischen der zweiten Schwerpunktwellenlänge 31, die hier 1040 nm ist, und der ersten Schwerpunktlänge 31, die hier 405 nm ist, besteht ein Farblängsfehler. In der Darstellung der Fig. 5 beispielsweise von etwa 1 mm. Im zweiten Wellenlängenbereich 26 um die zweite Schwerpunktwellenlänge 31 herum ist dieser Farblängsfehler jedoch, wie die in der Figur unten gezeigte Vergrößerung verdeutlicht, durch eine besondere Korrektur 28 des Systems 1 weitgehend konstant und bleibt innerhalb einer Farblängsfehlertoleranz 27, die im vergrößerten Ausschnitt gestrichelt eingezeichnet ist. Der vergrößerte Ausschnitt zeigt dabei den zweiten Wellenlängenbereich 26 mit der erreichten korrigierten Kennlinie 25. Zusätzlich ist die herkömmliche Kennlinie 24 so eingezeichnet, dass sie die korrigierte Kennlinie 25 schneidet. Dies dient dazu, um die unterschiedliche Steigung zu verdeutlichen. Die Kennlinie 25 ist durch die besondere Korrektur 28 des Systems gegenüber der unkorrigierten Kennlinie 24 weitestgehend horizontal, so dass sie innerhalb der Farblängsfehlertoleranz 27 bleibt. Die Kennlinie 24 hat diese Eigenschaft nicht. Würde man also ein System verwenden, dass die Kennlinie 24 hat, könnte man zwar den Farblängsfehler exakt für die zweite Schwerpunktwellenlänge ausgleichen. Man müsste dann allerdings eine Umfokussierung verwenden, die größer ist als die Umfokussierung 29. Dies könnte möglicherweise tolerierbar sein. Man hätte jedoch dann das Problem, dass innerhalb des Wellenlängenbereiches 26 um die zweite Schwerpunktwellenlänge die Kennlinie über die Farblängsfehlertoleranz 27 hinausginge, was zu einer axialen Aufweitung des Fokus führen würde. Dies wäre für eine nichtlineare Wechselwirkung (oder alternativ eine qualitativ hochwertige Augenbeobachtung) nicht mehr tolerabel.

Aus diesem Grund ist die Optik des Therapiesystems 1 korrigiert, also insbesondere die Fokussieroptik 10 und das Objektiv 11. Anstelle einer Kennlinie 24, die im zweiten Wellenlängenbereich 26 noch eine Steigung hätte, welche eine Variation des Farblängsfehlers zur Folge hätte, die weitaus größer wäre als die Farblängsfehlertoleranz 27, verläuft nun die Kennlinie 25 im Wesentlichen horizontal. Auf die absolute Ablage des Farblängsfehlers kommt es bei der Korrektur 28 nicht an. Vielmehr wird ein Farblängsfehler zwischen zweiter Schwerpunktwellenlänge 31 und erster Schwerpunktwellenlänge 30 bewusst in Kauf genommen. Aufgrund des im wesentlichen horizontalen Verlaufes der Kennlinie 25 - weil also die Schwankung des Farblängsfehlers im zweiten Wellenlängenbereich 26 innerhalb der Farblängsfehlertoleranz 27 bleibt - kann durch eine einfache Umfokussierung 29 der Farblängsfehler, der bei der zweiten Schwerpunktwellenlänge 31 vorhanden ist, ausgeglichen werden. Aufgrund der Korrektur 29, welche die Kennlinie 25 innerhalb der Farblängsfehlertoleranz 27 konstant hält, ist das Fokusvolumen axial nicht unzulässig aufgeweitet. Die axiale Fokusgüte bleibt weiterhin so, dass die nichtlineare Wechselwirkung erreicht wird.

Damit dies bei beiden Schwerpunktwellenlängen 30, 31 gegeben ist, ist die Farblängsfehlertoleranz 27 natürlich auch im Wellenlängenbereich um die erste

Schwerpunktwellenlänge 31 eingehalten. Sie ist in Fig. 5 zur einfacheren Darstellung nur für den Wellenlängenbereich 26 um die zweite Schwerpunktwellenlänge 30 eingezeichnet.

Die Kennlinie 25 des Therapiesystems 1 kann besonders einfach dadurch erreicht werden, dass die Optik des Therapiesystems für die erste Schwerpunktwellenlänge 30, also für 405 nm hinsichtlich einer üblichen Farblängsfehlerkorrektur ausgelegt wird. Für die zweite Schwerpunktwellenlänge 31 muss dann lediglich dafür gesorgt werden, dass die Konstanz der Kennlinie 25 auch im Wellenlängenbereich 26 um die zweite Schwerpunktwellenlänge 31 herum gegeben ist. Dies wird durch das Korrekturoptikglied 15a-15d erreicht, welches ausschließlich von dem zweiten Laserstrahl 6 bei der zweiten Schwerpunktwellenlänge 31 durchstrahlt wird. Die Ausgestaltung des Therapiesystems 1 mit dem Korrekturoptikglied 15a in der Pupillenebene 14 und in einem Abschnitt des Strahlengangs vor der Vereinigung an der xy-Scannereinrichtung 9 ist eine Möglichkeit, diese Korrektur und Konstanz der Kennlinie im Wellenlängenbereich 26 zu erreichen. Eine andere Möglichkeit besteht darin, ein Korrekturoptikglied 15b vorzusehen, das genau dann in eine Pupille des gemeinsamen Strahlengangs, beispielsweise in eine Pupille der Fokussieroptik 10 eingeschwenkt wird, wenn der zweite Laserstrahl 6 bei der zweiten Schwerpunktwellenlänge 31 aktiv ist. Eine weitere Option besteht darin, in einer gemeinsam durchstrahlten Pupille ein verstellbares Korrekturoptikglied 15c vorzusehen, dass hinsichtlich seiner Korrekturwirkung ein- und ausgeschaltet werden kann. Es wird genau dann auf Korrekturwirkung geschaltet, wenn der zweite Laserstrahl 6 durch das System läuft. Ebenfalls ist es möglich, das Korrekturoptikglied am Vereiniger 18 vorzusehen. Fig. 3 zeigt dazu exemplarisch eine Anordnung eines Korrekturoptikgliedes 15d, das ausschließlich auf den zweiten Laserstrahl 6 wirkt.

Fig. 4 zeigt eine mögliche Bauweise für das Korrekturoptikglied 15a, b, d, umfassend ein negatives Optikglied 22 und ein positives Optikglied 23. Optische Korrekturen zum Beeinflussen der Farblängsfehlerkennlinie sind dem Fachmann grundsätzlich bekannt.

Die Fokuslage und Fokusausdehnung wird aber nicht nur durch den Farblängsfehler Z beeinflusst, sondern auch durch den Farbquerfehler F. Dies ist insbesondere beim Therapiesystem 1 von Bedeutung, da es sich um ein scannendes Therapiesystem handelt, welches die Lage eines Fokus in einem Bildfeld lateral verstellt. Die xy-Scannereinrichtung 9 bewirkt, dass die Fokussieroptik 10 je nach lateraler Fokuslage in unterschiedlichen Ablagen zur optischen Achse durchstrahlt wird. Mit diesen Ablagen zeigen sich natürlich dadurch chromatische Farbquerfehler.

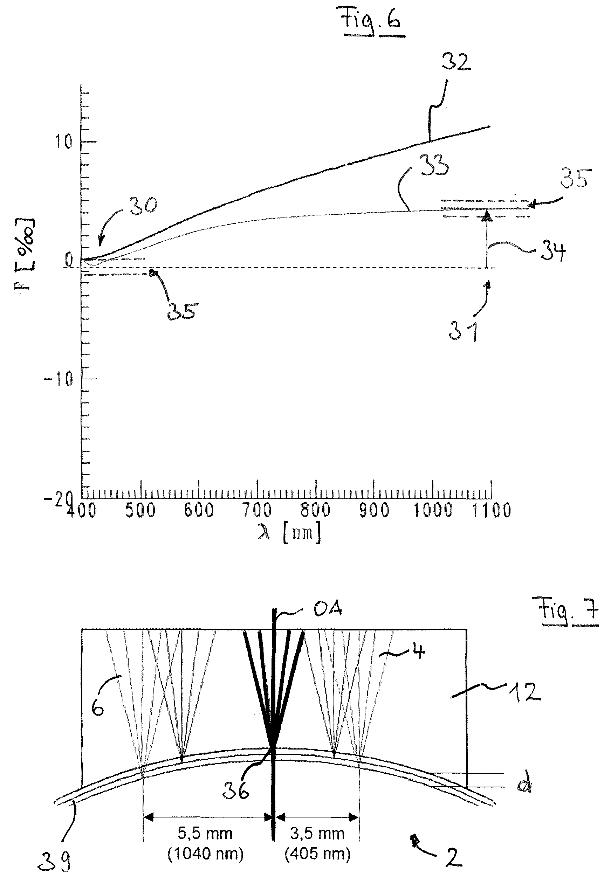

Fig. 6 zeigt die dazu vorgesehene Korrektur des Therapiesystems 1 und insbesondere der Fokussieroptik 10. Aufgetragen ist der spektrale Verlauf der Kennlinie für den Farbquerfehler F, der hier exemplarisch als relativer Farbquerfehler in ‰ aufgetragen ist.

Bei der ersten Schwerpunktwellenlänge 30 ist der Farbquerfehler 405 korrigiert. Sie ist somit die Bezugswellenlänge. Dies wäre auch für eine herkömmliche Kennlinie 32 der Fall. Die Korrektur ist weiter so ausgeführt, dass sich die Kennlinie 33 ergibt, die bei der zweiten Schwerpunktwellenlänge 31 im Wesentlichen konstant ist. Sie bleibt innerhalb einer Farbquerfehlertoleranz 35. Dies wäre für eine allein auf 405 nm bezogene Korrektur i.d.R. nicht der Fall. Die Fokussieroptik 10 ist hierfür besonders korrigiert. Der bei der zweiten Schwerpunktwellenlänge 31 vorhandene Farbquerfehler wirkt sich also in einer Ablenkwinkelmodifikation 34 aus. Durch eine entsprechend geänderte Ansteuerung der xy-Scannereinrichtung 9 kann der Farbquerfehler einfach ausgeglichen werden, wenn das Therapiesystem 1 bei der zweiten Schwerpunktwellenlänge 31, also mit dem zweiten Laserstrahl 6 arbeitet. Da der Farbquerfehler auch im zweiten Wellenlängenbereich innerhalb der Farbquerfehlertoleranz 35 bleibt, ist eine unzulässige Aufweitung des Fokusvolumens in lateraler Richtung, also quer zur optischen Achse, vermieden und im Fokus werden weiterhin zuverlässig nichtlineare Bearbeitungseffekte innerhalb des Gewebes erreicht. Es erübrigt sich zu sagen, dass auch bei der ersten Schwerpunktwellenlänge 30 der Farbquerfehler innerhalb der Farbquerfehlertoleranz 35 bleibt. Sie beträgt z. B. 0,5 µm absolut.

Die Kennlinie 33 erfordert keine perfekte Apochromatisierung der Fokussieroptik 10. Diese würde ja darin bestehen, dass der Farbquerfehler auch um die zweite Schwerpunktwellenlänge 31 herum verschwindet. Da diese Forderung nicht gestellt ist, sondern lediglich der Farbquerfehler sowohl im zweiten Wellenlängenbereich, der die zweite Schwerpunktwellenlänge 31 umgibt, als auch im ersten Wellenlängenbereich, der die erste Schwerpunktwellenlänge 30 umgibt, innerhalb der Farbquerfehlertoleranz bleiben muss, ist die optische Korrektur vereinfacht.

Fig. 7 zeigt die Auswirkung auf die Lage eines Fokus 36 in der Kornea 39 des Auges 2. Bei Einfall längs der optischen Achse OA wirkt sich der Farbquerfehler F nicht aus. Er spielt erst eine Rolle, wenn der Fokus 36 lateral in der Kornea 39 abgelenkt wird. Bei gleicher Auslenkung durch die Scanspiegel 20 ergibt sich je nach Wellenlänge eine unterschiedliche laterale Verschiebung des Fokus 36. Die zwei linken Strahlenbündel zeigen die Verhältnisse für den zweiten Laserstrahl 6 bei der zweiten Schwerpunktwellenlänge 31, die zwei rechten Strahlenbündel für den ersten Laserstrahl 4 bei der ersten Schwerpunktwellenlänge 30. Wie zu erkennen ist, ist bei gleichem Ablenkwinkel durch die Scanspiegel 20 eine unterschiedliche laterale Verschiebung des Fokus 36 in der Kornea 39 gegeben. Dies stellt sich aufgrund des vorhandenen Farbquerfehlers bei der zweiten Schwerpunktwellenlänge 31 ein. Durch eine passende Ablenkwinkelmodifikation 36 gleicht die Steuereinrichtung S diesen Unterschied aus, so dass der Fokus 36 wieder an der gewünschten Stelle in der Hornhaut liegt.

Fig. 7 zeigt weiter die Bedeutung der Umfokussierung zum Ausgleich des Farblängsfehlers. Die Dicke d der Hornhaut beträgt 0,5 mm. Ohne Umfokussierung hätte der Farblängsfehler zwischen zweiter und erster Schwerpunktwellenlänge 31, 30 einen Wert von 1 mm. Dieser Wert wäre größer als die Dicke d der Hornhaut 39, so dass der Fokus 36 bei einer der beiden Wellenlängen unerwünscht gänzlich außerhalb der Hornhaut 39 läge. Durch die Umfokussierung 29 ist dies verhindert. Die Umfokussierung 29 kann durch eine entsprechende Ausgestaltung der Steuereinrichtung S ausgeführt sein, die die Ansteuerung des betroffenen z-Scanners 7, 8 mit einem Offset versieht, um die Umfokussierung 29 zu bewirken und den Farblängsfehler auszugleichen. Alternativ oder zusätzlich kann ein entsprechendes Optikglied verwendet werden, das die axiale Lage verändert. Dieses Optikglied kann in Ausführungsformen mit dem Korrekturoptikglied 15a-d kombiniert oder in diesem vorgesehen sein.

Die Fig. 5 und 6 zeigen Verhältnisse, in denen der Farblängsfehler bzw. der Farbquerfehler für eine Wellenlänge, in den gezeigten Ausführungsformen für die erste Schwerpunktwellenlänge 30, korrigiert ist. Die erste Schwerpunktwellenlänge ist also die eingangs genannte Bezugswellenlänge. Dies ist jedoch nicht zwingend erforderlich. Es sind auch Kennlinien 25, 33 möglich, die an beiden Schwerpunktwellenlängen 30, 31 einen von Null verschiedenen Farblängsfehler Z und/oder Farbquerfehler F also eine ganz andere Bezugswellenlänge haben. Dies ist völlig unschädlich, solange die entsprechenden Fehler die vorgeschriebenen Fehlertoleranzbereiche in den entsprechenden Wellenlängenbereichen um die Schwerpunktwellenlängen 30, 31 nicht überschreiten.

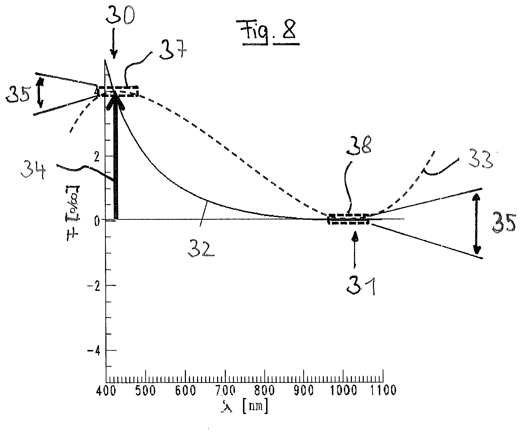

Fig. 8 zeigt eine exemplarisch entsprechende Ausgestaltung hinsichtlich des Farbquerfehlers F. Dieser ist hier bei der zweiten Schwerpunktwellenlänge 31 Null und bei der ersten Schwerpunktwellenlänge 30 von Null verschieden. Es ist jedoch wiederum darauf geachtet, dass innerhalb der Wellenlängenbereiche, die hier mit 37 und 38 eingetragen sind, der Farbquerfehler die Farbquerfehlertoleranz 35 nicht überschreitet.

Die Korrektur des Therapiesystems 1 ist also grundsätzlich so gestaltet, dass sowohl der Farblängsfehler als auch der Farbquerfehler in den Wellenlängenbereichen, welche sich aufgrund der Pulslänge um die Schwerpunktwellenlängen 30, 31 herum ergeben, lokale Extremata hat, die so flach ausgebildet sind, dass in den Wellenlängenbereichen die entsprechenden Toleranzen nicht überschritten werden und zuverlässig die nichtlineare Wechselwirkung im Fokus eintritt. Die Korrektur ist dadurch sehr viel einfacher, als wenn bei beiden Schwerpunktwellenlängen 30, 31 und innerhalb der sie umgebenden Wellenlängenbereiche verschwindende Farblängsfehler und Farbquerfehler gefordert würden.

Hinsichtlich des Farblängsfehlers ist die Einstellung des Nullwertes letztlich eine Skalierung. Es ist bei jeder Kurve möglich, den z-Wert bei einer der beiden Schwerpunktwellenlängen als Bezugsebene, d.h. als Nullebene der Fokustiefenangabe zu definieren. Letztlich ist der Farblängsfehler physikalisch gesehen ja nur eine Änderung der Fokalebene beim Wechsel zwischen zwei Wellenlängen, hier den Schwerpunktwellenlängen 30, 31.

Zusätzlich zu chromatischen Fehlern hat jedes abbildende System auch monochromatische Fehler. Hier ist das Therapiesystem in Ausführungsform c so gestaltet, dass die monochromatischen Fehler nur für eine der beiden Wellenlängen für den Strahlengang von der Laserquelle bis zum Auge ausgeglichen sind. Für die andere Wellenlänge wird ein Ausgleichselement in den Teil des Strahlengangs eingefügt, der nur von dieser Wellenlänge durchstrahlt wird, oder es wird ein Ausgleichselement in den gemeinsamen Strahlengang eingeschwenkt, wenn die andere Wellenlänge aktiv ist oder es wird ein verstellbares Ausgleichselement im gemeinsamen Strahlengang aktiviert bzw. geeignet eingestellt, wenn die andere Wellenlänge aktiv ist. In einer besonders zweckmäßigen Ausführungsform wird das Ausgleichselement mit dem Korrekturoptikglied kombiniert.

## Patentansprüche

1. System zur Augentherapie durch Bearbeitung von Gewebe (39) mittels nichtlineare Wechselwirkung mit Therapiestrahlung (4, 6), wobei das System (1) aufweist:
- eine Lasereinrichtung (3, 5), welche die Therapiestrahlung (4, 6) bereitstellt,
- eine Fokussiereinrichtung (10), die ein Bildfeld, das in einem Therapievolumen liegt, aufweist und die Therapiestrahlung (4, 6) derart zur einem Fokus (36) im Bildfeld bündelt, dass innerhalb des Gewebe (38) die Bearbeitung durch nichtlineare Wechselwirkung erzeugbar ist,
- eine der Fokussiereinrichtung (10) vorgeordnete xy-Scannereinrichtung (9) zur lateralen Verschiebung des Fokus (36) im Bildfeld,
- eine z-Scannereinrichtung (7, 8), die eine Tiefenlage des Fokus (36) im Therapievolumen verstellt, und
- eine Steuereinrichtung (S), welche die xy-Scannereinrichtung (9) und z-Scannereinrichtung (7, 8) ansteuert,
wobei
- die Lasereinrichtung (3, 5) als Therapiestrahlung eine erste Kurzpulsstrahlung (4) bei einer ersten Schwerpunktwellenlänge (30) und eine zweite Kurzpulsstrahlung (6) bei einer zweiten Schwerpunktwellenlänge (31), die von der ersten Schwerpunktwellenlänge (30) um mind. 300 nm beabstandet ist, bereitstellt, wobei die erste Kurzpulsstrahlung (4) aufgrund einer Pulslänge einen ersten Wellenlängenbereich (37) um die erste Schwerpunktwellenlänge (30) abdeckt und die zweite Kurzpulsstrahlung (6) aufgrund einer Pulslänge einen zweiten Wellenlängenbereich (38) um die zweite Schwerpunktwellenlänge (31) abdeckt, wobei
- das System (1) entweder mit der ersten Kurzpulsstrahlung (4) oder der zweiten Kurzpulsstrahlung (6) arbeitet, **dadurch gekennzeichnet, dass**
- das System (1) hinsichtlich Farblängsfehler (Z) und Farbquerfehler (F) derart ausgebildet ist, dass
-- das System (1) bei mindestens einer der beiden Schwerpunktwellenlängen (30, 31) einen Farblängsfehler (Z) hat, wobei eine spektrale Kennlinie (25) des Farblängsfehlers (Z) in beiden Wellenlängenbereichen (30, 31) jeweils im Rahmen einer Farblängsfehlertoleranz (27) konstant ist, und
-- das System (1) bei mindestens einer der beiden Schwerpunktwellenlängen (30, 31) einen Farbquerfehler hat, wobei eine spektrale Kennlinie (33) des Farbquerfehlers (F) in beiden Wellenlängenbereichen (30, 31) jeweils im Rahmen einer Farbquerfehlertoleranz (35) konstant ist, und
- die Steuereinrichtung (S) ausgebildet ist, die xy-Scannereinrichtung (9) so anzusteuern, dass der Farbquerfehler durch eine entsprechende Ablenkfunktion (34) für die laterale Verschiebung des Fokus (36) ausgeglichen ist.

2. System nach Anspruch 1, wobei eine Wellenlänge im ersten Wellenlängenbereich als Bezugswellenläge für den Farblängsfehler (Z) verwendet wird und die Steuereinrichtung (S) ausgebildet ist, die z-Scannereinrichtung (7, 8) so anzusteuern, dass bei Betrieb mit der zweiten Kurzpulsstrahlung (4) der Farblängsfehler (Z) durch einen entsprechenden Offset der Tiefenlage der Fokus (36) ausgeglichen ist.

3. System nach Anspruch 1 oder 2, wobei eine Wellenlänge im ersten Wellenlängenbereich als Bezugswellenläge für den Farblängsfehler (Z) verwendet wird und das System (1) ein nur auf die zweite Kurzpulsstrahlung (4) wirkendes oder nur bei Betrieb mit der zweiten Kurzpulsstrahlung (4) aktiviertes Umfokussierungselement (15) aufweist, das den Farblängsfehler durch einen entsprechenden Offset der Tiefenlage der Fokus (36) ausgleicht.

4. System nach einem der obigen Ansprüche , das ein nur auf die zweite Kurzpulsstrahlung (4) wirkendes oder nur bei Betrieb mit der zweiten Kurzpulsstrahlung (4) aktiviertes Umfokussierungselement (15) aufweist, welches bewirkt, dass die Kennlinie (25) des Farblängsfehlers (Z) im zweiten Wellenlängenbereich (38) innerhalb der Farblängsfehlertoleranz (27) konstant ist.

5. System nach einem der obigen Ansprüche, das einen ersten Eingangsstrahlengang für die erste Kurzpulsstrahlung (4) und einen zweiten Eingangsstrahlengang für die zweite Kurzpulsstrahlung (6) und eine der Fokussierungseinrichtung (10) vorgeordnete Vereinigereinrichtung (18) aufweist, die die beiden Eingangsstrahlengänge zusammenführt oder zwischen den beiden Eingangsstrahlengänge umschaltet, wobei der erste Eingangsstrahlengang und die Fokussierungseinrichtung (10) gemeinsam die konstante, spektrale Kennlinie (25) des Farblängsfehlers (F) im ersten Wellenlängenbereich (37) bewirken, wobei im Strahlengang nach der Vereinigereinrichtung (18) ein von der Steuereinrichtung angesteuertes Korrekturglied (15b, 15c) angeordnet ist, das hinsichtlich einer Korrekturwirkung deaktivierbar ist und bei aktivierter Korrekturwirkung die im Rahmen einer Farblängsfehlertoleranz (27) konstante, spektrale Kennlinie (25) des Farblängsfehlers (Z) im zweiten Wellenlängenbereich (38) bewirkt, und wobei die Steuereinrichtung (S) das Korrekturglied (15b, 15c) hinsichtlich dessen Korrekturwirkung aktiviert, wenn die zweite Kurzpulsstrahlung (6) durch das System (1) läuft, und das Korrekturglied (15a, 15b) hinsichtlich dessen Korrekturwirkung deaktiviert, wenn die erste Kurzpulsstrahlung (4) durch das System (1) läuft.

6. System nach Anspruch 5, wobei die Fokussierungsoptik (10) der xy-Scannereinrichtung (9) nachgeordnet ist und entweder das Korrekturglied (15d) zum Aktivieren in eine Pupille der Fokussierungsoptik (10) schaltbar, bevorzugt schwenkbar, ist, oder das Korrekturglied (15b) in einer Pupille der Fokussierungsoptik (10) angeordnet ist und als verstellbares Optikelement (15b) ausgebildet ist, insbesondere als Variolinse oder MEMS-Array.

7. System nach einem der Ansprüche 1 bis 4, das einen ersten Eingangsstrahlengang für die erste Kurzpulsstrahlung (4) und einen zweiten Eingangsstrahlengang für die zweite Kurzpulsstrahlung (6) und eine der Fokussierungseinrichtung (10) vorgeordnete Vereinigereinrichtung (18) aufweist, die die beiden Eingangsstrahlengänge zusammenführt oder zwischen den beiden Eingangsstrahlengängen umschaltet, wobei der erste Eingangsstrahlengang und die Fokussierungseinrichtung (10) gemeinsam die konstante, spektrale Kennlinie (25) des Farblängsfehlers (Z) im ersten Wellenlängenbereich (37) bewirken, wobei die Vereinigereinrichtung (18) ein nur auf die zweite Kurzpulsstrahlung (6) wirkendes Korrekturglied (15d) aufweist, das die im Rahmen einer Farblängsfehlertoleranz (27) konstante, spektrale Kennlinie (25) des Farblängsfehlers (Z) im zweiten Wellenlängenbereich (38) bewirkt.

8. System nach einem der Ansprüche 1 bis 4, das einen ersten Eingangsstrahlengang für die erste Kurzpulsstrahlung (4) und einen zweiten Eingangsstrahlengang für die zweite Kurzpulsstrahlung (6) und eine der Fokussierungseinrichtung (10) vorgeordnete Vereinigereinrichtung (18) aufweist, die die beiden Eingangsstrahlengänge zusammenführt oder zwischen den beiden Eingangsstrahlengängen umschaltet, wobei der erste Eingangsstrahlengang und die Fokussierungseinrichtung (10) gemeinsam die konstante, spektrale Kennlinie (27) des Farblängsfehlers (Z) im ersten Wellenlängenbereich (37) bewirken, und wobei der zweite Eingangsstrahlengang ein Korrekturglied (15a, 16) aufweist, das die im Rahmen der Farblängsfehlertoleranz (27) konstante, spektrale Kennlinie (25) des Farblängsfehlers (Z) im zweiten Wellenlängenbereich (38) bewirkt.

9. System nach Anspruch 8, dessen z-Scannereinrichtung (9) einen zweiten z-Scanner (8) für die zweite Kurzpulsstrahlung (6), der im zweiten Eingangsstrahlengang angeordnet ist, und einen ersten z-Scanner (7) für die erste Kurzpulsstrahlung (4), der im ersten Eingangsstrahlengang angeordnet ist, aufweist, wobei das Korrekturglied (16) im zweiten z-Scanner (8) angeordnet ist.

10. System nach Anspruch 9, dessen zweiter z-Scanner (8) als Teleskop mit einem beweglichen (17) und einem festen Optikglied (16) ausgebildet ist, wobei das feste Optikglied (16) zugleich als das Korrekturglied ausgebildet ist.

11. System nach einem der obigen Ansprüche soweit rückbezogen auf Anspruch 2, wobei das Korrekturglied (15a-d, 16) zugleich als Umfokussierungselement ausgebildet ist.

12. System nach einem der obigen Ansprüche, wobei die Fokussiereinrichtung (10) für einen der beiden Wellenlängenbereiche hinsichtlich monochromatischer Fehler korrigiert ist und im anderen Wellenlängenbereich einen monochromatischen Restfehler hat und in einem nur von der Strahlung im anderen Wellenlängenbereich durchstrahlten Strahlengang eine Ausgleichsoptik (15a,15d) angeordnet ist, die den monochromatischen Restfehler ausgleicht.

13. System nach Anspruch 12 in Kombination mit einem der Ansprüche 5 bis 11, wobei Korrekturglied und Ausgleichsoptik zu einem Korrekturoptikglied (15a, 15d) zusammengefasst sind.

14. System nach einem der obigen Ansprüche, wobei einer der beiden Schwerpunktwellenlängen nicht über 420 nm, bevorzugt zwischen 380 und 420 nm liegt, und die andere der beiden Schwerpunktwellenlängen nicht unter 730 nm, bevorzugt zwischen 1030 und 1060 nm, liegt und/oder wobei die beiden Wellenlängenbereiche jeweils nicht breiter sind als 30 nm, bevorzugt nicht breiter als 15 nm.

15. System nach einem der obigen Ansprüche, wobei ein z-Hub der z-Scannereinrichtung (7, 8) mindestens 1 mm, bevorzugt mindestens 3 mm, weiter bevorzugt mindestens 5 mm und besonders bevorzugt mindestens 13 mm beträgt.

## Claims

1. A system for therapy of the eye by treating tissue (39) by means of a nonlinear interaction with therapy radiation (4, 6), the system (1) comprising:
- a laser device (3, 5), which provides the therapy radiation (4, 6),
- a focusing device (10), which comprises an image field lying in a therapy volume and which focuses the therapy radiation (4, 6) onto a focus (36) in the image field in such a way that the treatment is producible by means of a nonlinear interaction within the tissue (38),
- an xy-scanner device (9), disposed upstream of the focusing device (10), for the lateral displacement of the focus (36) in the image field,
- a z-scanner device (7, 8), which adjusts a depth position of the focus (36) in the therapy volume, and
- a control device (S), which actuates the xy-scanner device (9) and the z-scanner device (7, 8),
wherein
- the laser device (3, 5) provides, as therapy radiation, a first short-pulse radiation (4) at a first centroid wavelength (30) and a second short-pulse radiation (6) at a second centroid wavelength (31), which is spaced apart from the first centroid wavelength (30) by at least 300 nm, wherein the first short-pulse radiation (4) covers a first wavelength range (37) about the first centroid wavelength (30) on account of a pulse length and the second short-pulse radiation (6) covers a second wavelength range (38) about the second centroid wavelength (31) on account of a pulse length, wherein
- the system (1) operates either with the first short-pulse radiation (4) or the second short-pulse radiation (6), **characterized in that**
- the system (1) is embodied in respect of longitudinal chromatic aberrations (Z) and transverse chromatic aberrations (F) in such a way that
-- the system (1) has a longitudinal chromatic aberration (Z) at at least one of the two centroid wavelengths (30, 31), wherein a spectral characteristic (25) of the longitudinal chromatic aberration (Z) in both wavelength ranges (30, 31) is constant in each case within the scope of a longitudinal chromatic aberration tolerance (27), and
-- the system (1) has a transverse chromatic aberration at at least one of the two centroid wavelengths (30, 31), wherein a spectral characteristic (33) of the transverse chromatic aberration (F) in both wavelength ranges (30, 31) is constant in each case within the scope of a transverse chromatic aberration tolerance (35), and
- the control device (S) is embodied to actuate the xy-scanner device (9) in such a way that the transverse chromatic aberration is compensated by a corresponding deflection function (34) for the lateral displacement of the focus (36).

2. The system as claimed in claim 1, wherein a wavelength in the first wavelength range is used as a reference wavelength for the longitudinal chromatic aberration (Z) and the control device (S) is embodied to actuate the z-scanner device (7, 8) in such a way that, during operation with the second short-pulse radiation (4), the longitudinal chromatic aberration (Z) is compensated by a corresponding offset in the depth position of the focus (36).

3. The system as claimed in claim 1 or 2, wherein a wavelength in the first wavelength range is used as a reference wavelength for the longitudinal chromatic aberration (Z) and the system (1) comprises a refocusing element (15), which only acts on the second short-pulse radiation (4) or which is only activated during operation with the second short-pulse radiation (4), said refocusing element compensating the longitudinal chromatic aberration by a corresponding offset in the depth position of the focus (36).

4. The system as claimed in any one of the preceding claims, comprising a refocusing element (15), which only acts on the second short-pulse radiation (4) or which is only activated during operation with the second short-pulse radiation (4), said refocusing element causing the characteristic (25) of the longitudinal chromatic aberration (Z) to be constant within the longitudinal chromatic aberration tolerance (27) in the second wavelength range (38).

5. The system as claimed in any one of the preceding claims, comprising a first input beam path for the first short-pulse radiation (4) and a second input beam path for the second short-pulse radiation (6) and a unifier device (18) disposed upstream of the focusing device (10), said unifier device merging the two input beam paths or switching between the two input beam paths, wherein the first input beam path and the focusing device (10) together cause the constant spectral characteristic (25) of the longitudinal chromatic aberration (F) in the first wavelength range (37), wherein a correction member (15b, 15c) that is actuated by the control device is disposed downstream of the unifier device (18) in the beam path, said correction member being deactivatable in view of a corrective effect and said correction member causing the spectral characteristic (25) of the longitudinal chromatic aberration (Z) in the second wavelength range (38), said spectral characteristic being constant within the scope of a longitudinal chromatic aberration tolerance (27), when the corrective effect is activated, and wherein the control device (S) activates the correction member (15b, 15c) in respect of its corrective effect when the second short-pulse radiation (6) passes through the system (1) and deactivates the correction member (15a, 15b) in respect of its corrective effect when the first short-pulse radiation (4) passes through the system (1).

6. The system as claimed in claim 5, wherein the focusing optical unit (10) is disposed downstream of the xy-scanner device (9) and the correction member (15d) is either switchable, preferably pivotable, for activation in a pupil of the focusing optical unit (10) or the correction member (15b) is arranged in a pupil of the focusing optical unit (10) and embodied as an adjustable optical element (15b), in particular as a varifocal lens or MEMS array.

7. The system as claimed in any one of claims 1 to 4, comprising a first input beam path for the first short-pulse radiation (4) and a second input beam path for the second short-pulse radiation (6) and a unifier device (18) disposed upstream of the focusing device (10), said unifier device merging the two input beam paths or switching between the two input beam paths, wherein the first input beam path and the focusing device (10) together cause the constant spectral characteristic (25) of the longitudinal chromatic aberration (Z) in the first wavelength range (37), wherein the unifier device (18) comprises a correction member (15d), which only acts on the second short-pulse radiation (6) and which causes the spectral characteristic (25) of the longitudinal chromatic aberration (Z) in the second wavelength range (38), said spectral characteristic being constant within the scope of a longitudinal chromatic aberration tolerance (27).

8. The system as claimed in any one of claims 1 to 4, comprising a first input beam path for the first short-pulse radiation (4) and a second input beam path for the second short-pulse radiation (6) and a unifier device (18) disposed upstream of the focusing device (10), said unifier device merging the two input beam paths or switching between the two input beam paths, wherein the first input beam path and the focusing device (10) together cause the constant spectral characteristic (27) of the longitudinal chromatic aberration (Z) in the first wavelength range (37), and wherein the second input beam path comprises a correction member (15a, 16), which causes the spectral characteristic (25) of the longitudinal chromatic aberration (Z) in the second wavelength range (38), said spectral characteristic being constant within the scope of the longitudinal chromatic aberration tolerance (27).

9. The system as claimed in claim 8, the z-scanner device (9) of which comprising a second z-scanner (8), which is arranged in the second input beam path, for the second short-pulse radiation (6) and a first z-scanner (7), which is arranged in the first input beam path, for the first short-pulse radiation (4), wherein the correction member (16) is arranged in the second z-scanner (8).

10. The system as claimed in claim 9, the second z-scanner (8) of which is embodied as a telescope with a movable (17) and a fixed optical member (16), wherein the fixed optical member (16) is embodied as the correction member at the same time.

11. The system as claimed in any one of the preceding claims, provided it refers back to claim 2, wherein the correction member (15a-d, 16) is embodied as the refocusing element at the same time.

12. The system as claimed in any one of the preceding claims, wherein the focusing device (10) is corrected in respect of monochromatic aberrations for one of the two wavelength ranges and said focusing device has a residual monochromatic aberration in the other wavelength range and a compensation optical unit (15a, 15d), which compensates the residual monochromatic aberration, is arranged in a beam path only passed through by the radiation in the other wavelength range.

13. The system as claimed in claim 12 in combination with any one of claims 5 to 11, wherein the correction member and the compensation optical unit are combined to form a correction optical member (15a, 15d).

14. The system as claimed in any one of the preceding claims, wherein one of the two centroid wavelengths does not lie over 420 nm, preferably lying between 380 and 420 nm, and the other of the two centroid wavelengths does not lie under 730 nm, preferably lying between 1030 and 1060 nm and/or wherein the two wavelength ranges are each no wider than 30 nm, preferably no wider than 15 nm.

15. The system as claimed in any one of the preceding claims, wherein a z-travel of the z-scanner device (7, 8) is at least 1 mm, preferably at least 3 mm, further preferably at least 5 mm and particularly preferably at least 13 mm.

## Revendications

1. Système de thérapie oculaire par traitement de tissu (39) au moyen d'une interaction non linéaire avec un rayonnement thérapeutique (4, 6), le système (1) comprenant :
- un dispositif laser (3, 5) fournissant le rayonnement thérapeutique (4, 6),
- un dispositif de focalisation (10) qui présente un champ d'image situé dans un volume thérapeutique et focalise le rayonnement thérapeutique (4, 6) en un foyer (36) dans le champ d'image de telle sorte qu'à l'intérieur du tissu (38) le traitement par interaction non linéaire peut être généré,
- un dispositif de balayage XY (9) placé en amont du dispositif de focalisation (10) pour un déplacement latéral du foyer (36) dans le champ d'image,
- un dispositif de balayage Z (7, 8) ajustant une profondeur du foyer (36) dans le volume thérapeutique, et
- un dispositif de commande (S) pilotant le dispositif de balayage XY (9) et le dispositif de balayage Z (7, 8), dans lequel
- le dispositif laser (3, 5) fournit comme rayonnement thérapeutique un premier rayonnement à impulsions courtes (4) sur une première longueur d'onde dominante (30) et un deuxième rayonnement à impulsions courtes (6) sur une deuxième longueur d'onde dominante (31) espacée d'au moins 300 nm de la première longueur d'onde dominante (30), le premier rayonnement à impulsions courtes (4) couvrant en raison d'une longueur d'impulsion une première plage de longueurs d'onde (37) autour de la longueur d'onde dominante (30) et le deuxième rayonnement à impulsions courtes (6) couvrant en raison d'une longueur d'impulsion une deuxième plage de longueurs d'onde (38) autour de la deuxième longueur d'onde dominante (31), dans lequel
- le système (1) fonctionne soit avec le premier rayonnement à impulsions courtes (4), soit avec le deuxième rayonnement à impulsions courtes (6),
**caractérisé en ce que**,
- concernant les aberrations chromatiques longitudinales (Z) et les aberrations chromatiques latérales (F), le système (1) est réalisé de telle sorte que
-- le système (1) présente sur au moins l'une des deux longueurs d'onde dominantes (30, 31) une aberration chromatique longitudinale (Z), une courbe caractéristique spectrale (25) de l'aberration chromatique (Z) étant constante respectivement dans la limite d'une tolérance aux aberrations chromatiques longitudinales (27), et
-- le système (1) présente sur au moins l'une des deux longueurs d'onde dominantes (30, 31) une aberration chromatique latérale, une courbe caractéristique spectrale (33) de l'aberration chromatique latérale (F) étant constante dans les deux plages de longueurs d'onde (30, 31) respectivement dans la limite d'une tolérance aux aberrations chromatiques latérales, et
- le dispositif de commande (S) est réalisé pour piloter le dispositif de balayage XY (9) de telle sorte que l'aberration chromatique latérale est compensée par une fonction de déviation correspondante (34) pour le déplacement latéral du foyer (36).

2. Système selon la revendication 1, dans lequel une longueur d'onde dans la première plage de longueurs d'onde est utilisée comme une longueur d'onde de référence pour l'aberration chromatique longitudinale (Z), et le dispositif de commande (S) est réalisé pour piloter le dispositif de balayage Z (7, 8) de telle sorte qu'en cas de fonctionnement avec le deuxième rayonnement à impulsions courtes (4), l'aberration chromatique longitudinale (Z) est compensée par un décalage correspondant de la profondeur du foyer (36).

3. Système selon la revendication 1 ou 2, dans lequel une longueur d'onde dans la première plage de longueurs d'onde est utilisée comme une longueur d'onde de référence pour l'aberration chromatique longitudinale (Z), et le système (1) présente un élément de refocalisation (15) n'agissant que sur le deuxième rayonnement à impulsions courtes (4) ou n'étant activé qu'en cas de fonctionnement avec le deuxième rayonnement à impulsions courtes (4) qui compense l'aberration chromatique longitudinale par un décalage correspondant de la profondeur du foyer (36).

4. Système selon l'une quelconque des revendications précédentes, comprenant un élément de refocalisation (15) n'agissant que sur le deuxième rayonnement à impulsions courtes (4) ou n'étant activé qu'en cas de fonctionnement avec le deuxième rayonnement à impulsions courtes (4) qui fait que la courbe caractéristique (25) de l'aberration chromatique longitudinale (Z) est constante dans la deuxième plage de longueurs d'onde (38) dans la limite de la tolérance aux aberrations chromatiques longitudinales (27).

5. Système selon l'une quelconque des revendications précédentes, comprenant un premier trajet optique d'entrée pour le premier rayonnement à impulsions courtes (4) et un deuxième trajet optique d'entrée pour le deuxième rayonnement à impulsions courtes (6) et un dispositif de concentration (18), placé en amont du dispositif de focalisation (10), qui concentre les deux trajets optiques d'entrée ou commute entre les deux trajets optiques d'entrée, dans lequel le premier trajet optique d'entrée et le dispositif de focalisation (10) causent ensemble la courbe caractéristique spectrale constante (25) de l'aberration chromatique longitudinale (F) dans la première plage de longueurs d'onde (37), dans lequel sur le trajet optique après le dispositif de concentration (18) est disposé un élément correcteur (15b, 15c) piloté par le dispositif de commande qui peut être désactivé quant à une action correctrice et cause en cas d'action correctrice activée la courbe caractéristique spectrale constante (25) dans la limite d'une tolérance aux aberrations chromatiques longitudinales (27) de l'aberration chromatique longitudinale (Z) dans la deuxième plage de longueurs d'onde (38), et dans lequel le dispositif de commande (S) active l'élément correcteur (15b, 15c) quant à son action correctrice lorsque le deuxième rayonnement à impulsions courtes (6) passe par le système (1), et désactive l'élément correcteur (15a, 15b) quant à son action correctrice lorsque le premier rayonnement à impulsions courtes (4) passe par le système (1).

6. Système selon la revendication 5, dans lequel l'optique de focalisation (10) est placée en aval du dispositif de balayage XY (9), et soit l'élément correcteur (15d) peut être commuté, de préférence amené à pivoter, pour une activation dans une pupille de l'optique de focalisation (10), soit l'élément correcteur (15b) est disposé dans une pupille de l'optique de focalisation (10) et est réalisé sous la forme d'un élément optique réglable (15b), en particulier comme un objectif variable ou un réseau MEMS.

7. Système selon l'une quelconque des revendications 1 à 4, comprenant un premier trajet optique d'entrée pour le premier rayonnement à impulsions courtes (4) et un deuxième trajet optique d'entrée pour le deuxième rayonnement à impulsions courtes (6) et un dispositif de concentration (18), placé en amont du dispositif de focalisation (10), qui concentre les deux trajets optiques d'entrée ou qui commute entre les deux trajets optiques d'entrée, dans lequel le premier trajet optique d'entrée et le dispositif de focalisation (10) causent ensemble la courbe caractéristique spectrale constante (25) de l'aberration chromatique longitudinale (Z) dans la première plage de longueurs d'onde (37), dans lequel le dispositif de concentration (18) présente un élément correcteur (15d) n'agissant que sur le deuxième rayonnement à impulsions courtes (6) qui cause la courbe caractéristique spectrale constante (25) de l'aberration chromatique longitudinale (Z) dans la deuxième plage de longueurs d'onde (38) dans la limite d'une tolérance aux aberrations chromatiques longitudinales (27).

8. Système selon l'une quelconque des revendications 1 à 4, comprenant un premier trajet optique d'entrée pour le premier rayonnement à impulsions courtes (4) et un deuxième trajet optique d'entrée pour le deuxième rayonnement à impulsions courtes (6) et un dispositif de concentration (18), placé en amont du dispositif de focalisation (10), qui concentre les deux trajets optiques d'entrée ou commute entre les deux trajets optiques d'entrée, dans lequel le premier trajet optique d'entrée et le dispositif de focalisation (10) causent ensemble la courbe caractéristique spectrale constante (27) de l'aberration chromatique longitudinale (Z) dans la première plage de longueurs d'onde (37), et dans lequel le deuxième trajet optique d'entrée présente un élément correcteur (15a, 16) qui cause la courbe caractéristique spectrale constante (25) dans la limite de la tolérance aux aberrations chromatiques longitudinales (27) de l'aberration chromatique longitudinale (Z) dans la deuxième plage de longueurs d'onde (38).

9. Système selon la revendication 8, dont le dispositif de balayage Z (9) présente un deuxième scanner Z (8) pour le deuxième rayonnement à impulsions courtes (6), disposé sur le deuxième trajet optique d'entrée, et un premier scanner Z (7) pour le premier rayonnement à impulsions courtes (4), disposé sur le premier trajet optique d'entrée, l'élément correcteur (16) étant disposé dans le deuxième scanner Z (8).

10. Système selon la revendication 9, dont le deuxième scanner Z (8) est réalisé sous forme de télescope avec un élément optique mobile (17) et un élément optique fixe (16), l'élément optique fixe (16) étant réalisé en même temps comme l'élément correcteur.

11. Système selon l'une quelconque des revendications précédentes, dans la mesure où elle dépend de la revendication 2, dans lequel l'élément correcteur (15a à d, 16) est réalisé en même temps comme l'élément de refocalisation.

12. Système selon l'une quelconque des revendications précédentes, dans lequel le dispositif de focalisation (10) pour l'une des deux plages de longueurs d'onde est corrigé quant à une aberration monochromatique et présente dans l'autre plage de longueurs d'onde une aberration monochromatique résiduelle, et sur un trajet optique traversé uniquement par le rayonnement dans l'autre plage de longueurs d'onde est disposé une optique de compensation (15a, 15d) qui compense l'aberration monochromatique résiduelle.

13. Système selon la revendication 12 en combinaison avec l'une quelconque des revendications 5 à 11, dans lequel l'élément correcteur et l'optique de compensation sont regroupés en un élément optique correcteur (15a, 15d).

14. Système selon l'une quelconque des revendications précédentes, dans lequel l'une des deux longueurs d'onde dominantes n'est pas supérieure à 420 nm, est de préférence comprise entre 380 et 420 nm, et l'autre des deux longueurs d'onde dominantes n'est pas inférieure à 730 nm, est de préférence comprise entre 1030 et 1060 nm, et/ou dans lequel les deux plages de longueurs d'onde ne sont pas plus larges que 30 nm, respectivement, de préférence pas plus larges que 15 nm.

15. Système selon l'une quelconque des revendications précédentes, dans lequel une course Z du dispositif de balayage Z (7, 8) mesure au moins 1 mm, de préférence au moins 3 mm, de plus grande préférence au moins 5 mm et de manière particulièrement préférée au moins 13 mm.
